# EUROPÄISCHE PATENTSCHRIFT

(11) **EP 3 410 105 B1**
(45) Veröffentlichungstag und Bekanntmachung des Hinweises auf die Patenterteilung: **30.06.2021**
(21) Anmeldenummer: 18173488.0
(22) Anmeldetag: 22.05.2018
(51) Int. Cl.: G01N 24/08, G01R 33/44

(54) **SCHNELLES VERFAHREN ZUR BESTIMMUNG DER POSITION EINES FERROMAGNETISCHEN PARTIKELS ODER EINES BÜNDELS FERROMAGNETISCHER PARTIKEL MIT MRI-SYSTEMEN**
QUICK METHOD FOR DETERMINING THE POSITION OF A FERROMAGNETIC PARTICLE OR A BUNDLE OF FERROMAGNETIC PARTICLES WITH MRI SYSTEMS
PROCÉDÉ RAPIDE DE DÉTERMINATION DE LA POSITION D'UNE PARTICULE FERROMAGNÉTIQUE OU D'UN FAISCEAU DE PARTICULES FERROMAGNÉTIQUES AU MOYEN DES SYSTÈMES IRM

(30) Priorität: 02.06.2017 DE 102017209373
(43) Veröffentlichungstag der Anmeldung: 05.12.2018
(73) Patentinhaber: Bruker BioSpin MRI GmbH, 76275 Ettlingen (DE)
(72) Erfinder: Nauerth, Arno, 76872 Erlenbach (DE)
(74) Vertreter: Kohler Schmid Möbus Patentanwälte

(56) Entgegenhaltungen:
- DE-A1- 19 958 408
- DE-B3-102015 224 085
- US-B2- 8 948 841
- DODD S.J. ET AL.: "Improving Detection of Micron Size Magnetic Particles Using Linear Phase Ramps", PROCEEDINGS OF THE INTERNATIONAL SOCIETY FOR MAGNETIC RESONANCE IN MEDICINE,, 19TH ANNUAL MEETING & EXHIBITION, 7. Mai 2011 (2011-05-07), Seite 1710, XP040618356, Canada

## Beschreibung

Die Erfindung betrifft ein Verfahren zur Bestimmung der Position wenigstens eines ferromagnetischen Partikels in einem Objekt mittels eines MRI-Systems, wobei eine MRI-Messsequenz auf ein Messvolumen angewandt wird, in welchem sich das Partikel befindet.

Ein solches Verfahren ist bekannt geworden aus der US 7,962,194 B2 (=Referenz [1]).

### Hintergrund der Erfindung

Bildgebende Magnetresonanz(=MRI)-Verfahren werden vielfältig genutzt, um Bildinformationen von Strukturen zu erhalten. Dabei ist es möglich, Bildinformationen auch aus dem Inneren einer Struktur zu erhalten, ohne die Struktur zu beschädigen. Beispielsweise werden in klinischen Anwendungen Innenansichten der Körperteile von Menschen und Tieren mittels MRI-Verfahren abgebildet.

Ferromagnetische Partikel innerhalb eines Magnetfeldes erzeugen signifikante Störfelder, deren Ausdehnung ein Vielfaches der Größe des Partikels überschreiten. Solche Störfelder erscheinen in herkömmlichen MRI-Messsequenzen im Kern des Störfeldes als dunkle Flecken ("schwarze Löcher") und erzeugen nach außen hin Signalauslöschungen und Bildverzerrungen. Dies hat einerseits zur Folge, dass die nähere Umgebung des Partikels nicht bzw. nur ungenau durch bildgebende MRI-Verfahren wiedergegeben wird. Andererseits erweist sich die genaue Ortsbestimmung ferromagnetischer Partikel als schwierig und in der Regel sehr zeitaufwändig.

Bei der Nutzung von Mikromaschinen, Mikrorobotern oder Mikrokomponenten stellt sich allgemein das Problem des Verbringens an einen gewünschten Ort (Einsatzort). Für Mikroroboter sind eigene Antriebssysteme, etwa Laufbeine, bekannt geworden; solche Antriebssysteme sind jedoch teuer und schwierig zu konstruieren. Bei ferromagnetischen Partikeln besteht hingegen die Möglichkeit, durch einen Magnetfeldgradienten von außen eine Kraft auf sie auszuüben. Dadurch wird es möglich, das ferromagnetische Partikel berührungslos zu bewegen. Neben dem Bewegen ist es für die Verbringung an einen gewünschten Ort notwendig, den momentanen Ort des ferromagnetischen Partikels zu überprüfen, insbesondere um gegebenenfalls den momentanen Ort durch weitere Bewegungen zu korrigieren. Wenn ein magnetisches Partikel auf dem Weg zu seinem Einsatzort von einer Flüssigkeitsmatrix umgeben ist und durch diese Flüssigkeitsmatrix fortbewegt werden muss, kann mit dem MRI-System jedoch in vielen Fällen die Position des ferromagnetischen Partikels in einer Bildaufnahmesequenz nicht vernünftig bestimmt werden. Das ferromagnetische Partikel erscheint im Bild oftmals unscharf und ist daher in der Bildaufnahme nicht genau lokalisierbar oder gar aus dem Messvolumen verschwunden.

Die US 8,948,841 B2 (=Referenz [2]) beschreibt einer Verfahren zum Nachverfolgen eines magnetischen Objekts mittels eines MRI-Systems, wobei mittels Projektionen von magnetischen Isoflächen der Ort des magnetischen Objekts berechnet wird. Insbesondere sieht das Verfahren dieses Dokuments vor, Projektionen in entgegengesetzte Raumrichtungen zur Positionsbestimmung auszuwerten.

Um genügend Informationen für die Bestimmung der Position des ferromagnetischen Partikels zu erhalten, ist grundsätzlich eine Vielzahl von Einzelmessungen nötig, die in einer Messsequenz nacheinander abgearbeitet werden. Nach den Einzelmessungen wird jeweils eine gewisse Zeit abgewartet, um die Kernspins relaxieren zu lassen. Während der Einzelmessungen und der Relaxationszeiten wirken Kräfte auf das Partikel, insbesondere durch Gravitation, durch Strömungen in der Flüssigkeitsmatrix oder auch durch die ortskodierenden Gradientenschaltungen während den Einzelmessungen. Diese Kräfte versuchen das Partikel während der Messsequenz zu verschieben. Verschiebungen während der Messsequenz können die Informationen für die Bestimmung der Position des ferromagnetischen Partikels korrumpieren. Im schlimmsten Fall wandert das Partikel aus dem Messvolumen heraus.

Unter dem Oberbegriff "Positive Contrast MRI" findet man mehrere Beispiele, wie z.B. Ansammlungen von Stammzellen nachgewiesen werden können, welche mit ferromagnetischen Nanopartikeln behaftet sind. Sechs solcher Verfahren beschreibt und vergleicht Evert-jan P. A. Vonken in einem Artikel in J Magn Reson Imaging. 2013 August; 38(2): 344-357 (=Referenz [3]). In diesem Dokument werden unter anderem folgende Methoden offenbart:
WM (White Marker): Bei dieser Methode wird die Verschiebung des MR Signals im k-Raum genutzt, um ein sichtbares Signal zu erhalten. Dies wird durch Änderung der Bildgebungsgradienten erreicht.

SGM (Susceptibility Gradient Mapping) bedient sich rein rechnerischer Methoden, um die Suszeptibilitätsänderung in Voxeln nahe des Partikels im k-Raum zu verschieben.

IRON (Inversion Recovery with ON-resonant water Suppression): (siehe auch Referenz [4]).

FSX (Frequency selective excitation): Diese Methode bedient sich Off-Resonanz Anregungspulsen im Bildgebenden Teil der Messsequenz.

FLAPS (Fast Low Angle Positive contrast Steady-state free precession): Diese Methode verwendet den Phasenrotationswinkel zwischen einander folgenden RF Anregungen. In Anwesenheit einen Suszeptibilitätsmarkers ändert sich der Winkel mit der Magnetfeldänderung.

IDEAL (Iterative Decomposition of water and fat with Echo Asymmetry and Least-squares Estimation): Hier werden inter-Echo Phasenverschiebungen genutzt und mit der Bildnachbearbeitung kombiniert.

Ein weiteres Verfahren beschreibt Matthias Stuber in dem Artikel Magnetic Resonance in Medicine 58:1072-1077 (2007) (=Referenz [4]), worin er die Off-Resonanzeffekte von Spins nahe dem ferromagnetischen Partikel ausnutzt, indem er On-Resonanz- Spins sättigt und somit lediglich Spins in der Nähe der ferromagnetischen Partikel abbildet.

Die genannten Verfahren haben alle die Eigenschaft, dass sie eine recht lange Aufnahmezeit benötigen und zusätzlich eine hohe Pulsleistung applizieren.

### Aufgabe der Erfindung

Demgegenüber ist es Aufgabe der vorliegenden Erfindung, eine zuverlässigere und präzisere Bestimmung der Position eines ferromagnetischen Partikels oder eines Bündels ferromagnetischer Partikel in einem Objekt oder einer Flüssigkeitsmatrix zu ermöglichen, wobei diese Positionsbestimmung in sehr kurzer Zeit erfolgen und dabei eine möglichst geringe Pulsleistung appliziert werden soll.

### Kurze Beschreibung der Erfindung

Diese Aufgabe wird wird auf ebenso überraschend einfache wie wirkungsvolle Weise mit einem Verfahren gemäss Anspruch 1 gelöst. Das Verfahren dient der Bestimmung der Position wenigstens eines ferromagnetischen Partikels in einem Objekt mittels eines MRI-Systems, wobei eine MRI-Messsequenz auf ein Messvolumen angewandt wird, in welchem sich das Partikel befindet, wobei mindestens zwei Projektionen pro Raumrichtung aufgenommen werden, und wobei jeweils ein Lesegradient zur Aufnahme jeweils einer eindimensionalen Projektion geschaltet wird. Für die beiden Projektionen wird ein Parameter vorgewählt und dessen Wert bei der zweiten Projektion gegenüber der ersten geändert, wobei als zu ändernder Parameter die Echo-Zeit oder eine Amplitude des Lesegradienten vorgewählt wird, und im Falle der Änderung der Amplitude des Lesegradienten das Field of View beibehalten wird. Mit Hilfe der beiden Projektionen wird die räumliche Position des ferromagnetischen Partikels ermittelt.

Bei diesem Verfahren zur Positionsbestimmung von ferromagnetischen Partikeln, die im MRI nur als Artefakt sichtbar sind, wird die Feldstörung in der Umgebung des Partikels ausgenutzt, welche die Signale räumlich verschiebt. Diese Verschiebung kann man mit unterschiedlichen Messmethoden sichtbar machen, und dann aus der Differenz oder den Projektionen bzw. aus deren Superposition die genaue Position des Partikels bzw. eines Bündels ferromagnetischer Partikel sehr schnell, exakt und ohne großen technischen Aufwand zu bestimmen.

Die Verwendung von eindimensionalen Projektionen, gegebenenfalls sogar unter Verzicht auf den Einsatz von Ortskodierungsgradienten, macht das erfindungsgemäße Verfahren besonders einfach und außerordentlich schnell.

Bei diesem Verfahren ist es lediglich erforderlich, den Lesegradienten um einen Parameter zu ändern und somit zwei Projektionen pro Raumrichtung aufzunehmen. Die Feldstörung, die durch das ferromagnetische Partikel hervorgerufen wird, bewirkt einen schnelleren Zerfall des FID, was sich im Umkehrschluss auf die Ausleseparameter auswirkt. Daher kann die Störung mittels zweier Projektionen lokalisiert werden, wenn der besagte Parameter variiert wird.

Die Messsequenz besteht im einfachsten Fall lediglich im Einstrahlen eines Anregungspulses und dem Schalten eines Lesegradienten mit unterschiedlichen Ausleseparametern während des FID. Beim Spin-EchoVerfahren kommt noch zusätzlich ein zweiter Puls hinzu, der nicht 180° betragen muss, da bei relativ großen Partikeln mit einem Durchmesser von mindestens 1mm auch schon ein Flipwinkel von 1° für die gewünschte Projektion ausreichend sein kann. Eine weitere Ortskodierung ist für die eindimensionale Projektion nicht notwendig.

Das erfindungsgemäße Verfahren wird typischerweise als Betriebsverfahren auf einem MRI-System eingerichtet, wobei eine Steuereinrichtung so eingerichtet bzw. programmiert ist, dass mindestens zwei Projektionen pro Raumrichtung aufgenommen werden, wobei jeweils ein Lesegradient zur Aufnahme jeweils einer eindimensionalen Projektion geschaltet wird, wobei für jede der beiden Projektionen ein vorgewählter Parameter gemäss Anspruch 1 geändert wird und wobei mit Hilfe der beiden Projektionen die räumliche Position des ferromagnetischen Partikels ermittelt wird.

### Bevorzugte Varianten der Erfindung

Gemäss einer Variante des erfindungsgemässen Verfahrens ist vorgesehen, dass der vorgewählte, zu ändernde Parameter die Echo-Zeit ist. Dies gilt sowohl für die Spin-Echo-Zeit als auch für die Gradienten-Echo- Zeit.

Alternativ ist der vorgewählte, zu ändernde Parameter eine Amplitude des Lesegradienten bei gleichzeitiger Beibehaltung des Field of Views. Dies resultiert in einer geänderten Sweepwidth.

Vorteilhaft sind Ausführungsformen des erfindungsgemäßen Verfahrens, bei welchen jeweils zwei 1dimensionale Projektionsbilder in drei unterschiedlichen, vorzugsweise zueinander senkrechten, Raumrichtungen aufgenommen werden und daraus die räumliche Position des ferromagnetischen Partikels im 3dimensionalen Raum bestimmt wird. Dies ist dann besonders sinnvoll, wenn man a priori nicht weiß, wo sich das Partikel befindet. Man wird dann gewissermaßen die gesamte Umgebung "durchkämmen".

Bei weiteren Ausführungsformen kann es vorteilhaft sein, wenn die Position des ferromagnetischen Partikels durch Differenzbildung der aufgenommenen 1 dimensionalen Projektionsbilder oder durch Übereinanderlegen der aufgenommenen 1dimensionalen Projektionsbilder oder mittels Mustererkennung im Vergleich der aufgenommenen 1dimensionalen Projektionsbilder miteinander bestimmt wird.

Ganz besonders bevorzugt sind auch Ausführungsformen des erfindungsgemäßen Verfahrens, die sich dadurch auszeichnen, dass das ferromagnetische Partikel mit einem anderen NMR-aktiven Kern markiert wird, z. B. durch Beschichtung. Ein wesentlicher Vorteil hierbei ist, dass bei Aufnahme einer Projektion mit einer anderen Frequenz, die nicht identisch mit der Protonenfrequenz ist, eine wesentlich gleichmäßigere Umgebung um das Partikel herrscht. Wenn das unbeschichtete Partikel beispielsweise an der Darmwand eines biologischen Untersuchungsobjekts anliegt, so ist es möglich, dass in der Umgebung eine Gasblase ist, welche *keinen* 1H Kern enthält. Daraus resultiert dann eine sehr ungenaue Projektion. Dasselbe gilt auch beispielsweise für unterschiedliche benachbarte Gewebe (Knochen, Fettgewebe, Flüssigkeit, etc.). Wird das Partikel beispielsweise mit Teflon, also Polytetrafluorethen (PTFE), beschichtet, so ist die Umgebung des Partikels immer homogen mit 19F Kernen versehen.

Bei vorteilhaften Weiterbildungen dieser Ausführungsformen können als Markier-oder Beschichtungsmaterial spinaktive Kerne verwendet werden, vorzugsweise solche, die als Heteroatome in Kunststoffen zu finden sind, wie F in Teflon, Cl in PVC, N in Polyurethanen, insbesondere 6Li, 10B, 14N, 15N, 17O, 19F, 23Na, 29Si, 31P, 35CI, 113Cd, 195Pt. Vorteilhaft können Materialien eingesetzt werden, die zur Beschichtung dienen können, vorzugsweise Inertmaterialien wie etwa PVC, PTFE, Silikon (Polysiloxane), Polyphosphazene (31P), Polyurethane (15N), ggf. auch Polyether oder Polyester (17O). Die Wahl von Pt wäre zwar möglich, ist aber relativ teuer. Cd könnte ebenfalls verwendet werden, ist jedoch giftig.

Die MRI-Signale aus dem Messvolumen können mittels einer Spin-Echo Methode (z.B. MSME) oder mittels einer Gradienten-Echo Methode, insbesondere mittels des FLASH-Verfahrens, oder mittels des ZTE-Verfahrens oder mittels des UTE-Verfahrens oder mittels des SPI-Verfahrens erzeugt werden.

### Erfindungsgemäßes MRI-System

In den Rahmen der vorliegenden Erfindung fällt weiterhin ein MRI-System, umfassend einen Magneten zur Erzeugung eines homogenen Magnetfelds B₀ in einem Messvolumen, ein Gradientenspulensystem zur Erzeugung von -insbesondere auch ortskodierenden- Magnetfeldgradienten im Messvolumen, und ein Hochfrequenz-Anregungs- und Lesespulensystem zum Einstrahlen von Hochfrequenz-Pulsen in das Messvolumen und zum Auslesen des Messvolumens, das dadurch gekennzeichnet ist, dass das MRI-System eingerichtet ist zum Bestimmen der Position eines ferromagnetischen Partikels gemäß einem oben beschriebenen, erfindungsgemäßen Verfahren. Das MRI-System bzw. die Steuereinrichtung weist eine entsprechende Programmierung auf, und ermöglich auf einfache Weise eine schnelle und ziemlich genaue Positionskontrolle von ferromagnetischen Partikeln.

Weitere Vorteile der Erfindung ergeben sich aus der Beschreibung und der Zeichnung. Ebenso können die vorstehend genannten und die noch weiter ausgeführten Merkmale erfindungsgemäß jeweils einzeln für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

### Detaillierte Beschreibung der Erfindung und Zeichnung

Die Erfindung ist in der Zeichnung dargestellt und wird anhand von Ausführungsbeispielen näher erläutert. Es zeigen:
- Fig. 1: Feldstörungen, abgebildet durch unterschiedliche Messverfahren;
- Fig. 2: Feldstörung aufgenommen mit MSME;
- Fig. 3: Feldstörungen in XZ Ebene und der zugehörigen Projektion, aufgenommen mit MSME bei positiver und negativer Gradientenschaltung;
- Fig. 4a: Feldstörungen ausgelöst durch Magnetpartikel bei unterschiedlicher Gradientenechozeit (1ms und 2ms) sowie die jeweiligen 1D Projektionen;
- Fig. 4b: Massenzentrum der Differenz-Projektion;
- Fig. 5: Einfluss der Sweep width auf die Feldstörungen;
- Fig. 6: Lokalisierung eines Magnetpartikels unter Ausnutzung eines asymmetrischen Artefakts mit Hilfe entgegengesetzter Lesegradienten; und
- Fig. 7: eine schematische Darstellung eines MRI-Systems zur Durchführung des erfindungsgemäßen Verfahrens.

### Überblick über die Erfindung

Die vorliegende Erfindung betrifft ein Verfahren für eine verbesserte Positionsbestimmung eines ferromagnetischen Partikels in einem Objekt, vorzugsweise in einer Fluidmatrix mit einem Magnetresonanz-Imaging(=MRI)-System.

Diese Erfindungsaufgabe wird gelöst, indem in geeigneter Weise eindimensionale Projektionen erzeugt werden, die es erlauben, die Position eines ferromagnetischen Partikels oder einem Bündel ferromagnetischer Partikel zu bestimmen.

Dabei beruht die erfindungsgemäße Lösung auf der Erkenntnis, dass in der nächsten Umgebung des ferromagnetischen Partikels das Magnetfeld derart gestört ist, dass die dort befindlichen Kernspins in ihrer magnetischen Kodierung durch die Gradienten geändert sind. Weiterhin bewirkt die Feldstörung des Partikels eine stärkere Dephasierung der Spins in der nächsten Umgebung des Partikels und daher mit einer geringeren Intensität in der Projektion abgebildet werden. Als Folge dieser lokalen Feldstörung werden die Signale, die durch die dort befindlichen Kernspins erzeugt werden, räumlich deplatziert. In einer Projektion werden die Verschiebungen sichtbar bzw. messbar gemacht.

Gegenüber bekannten Verfahren zeichnet sich das erfindungsgemäße dadurch aus, dass MRI-Signale aus dem Messvolumen aufgenommen werden, die artifizielle räumliche Verschiebungen aufgrund von Magnetfeldstörungen in der Umgebung des ferromagnetischen Partikels aufweisen, welche durch die Anwesenheit des ferromagnetischen Partikels im NMR-Magnetfeld im Messvolumen hervorgerufen werden, und dass anschließend mit Hilfe dieser gemessenen räumlichen Verschiebungen in den MRI-Signalen die räumliche Position des ferromagnetischen Partikels ermittelt wird.

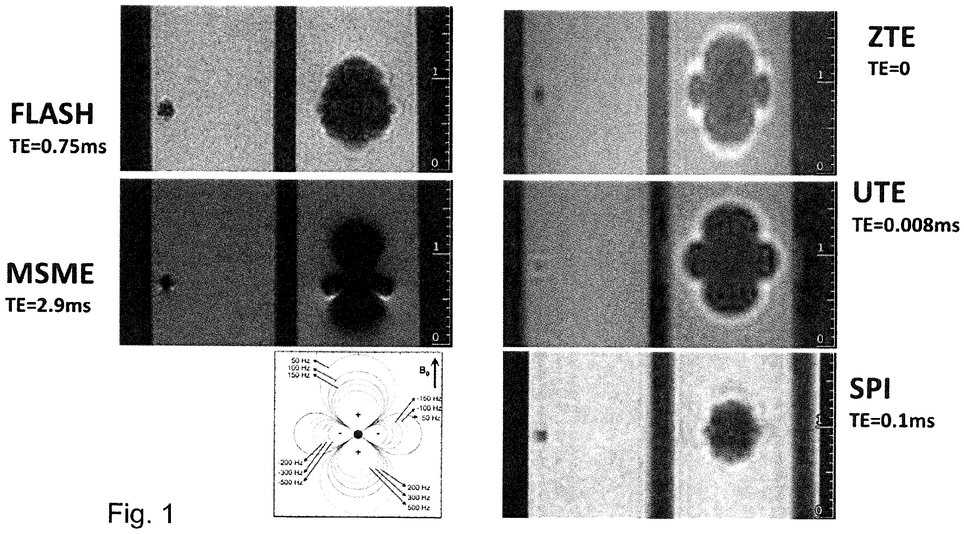

In **Fig. 1** sind mehrere MRI Aufnahmen zu sehen, wobei zwei ferromagnetische Partikel unterschiedlicher Größe in einer Gelmatrix vorgelegt sind. Auf der linken Seite befindet sich jeweils ein Probengefäß mit einem Partikel des Durchmessers 0,35mm, die rechte Seite der Aufnahme ist ein Probengefäß mit einem Partikel des Durchmessers 1mm. Es ist dargestellt, wie sich eine Feldstörung, die durch Magnetpartikel hervorgerufen wird, auf unterschiedliche Messmethoden im MRI auswirken. In jedem Fall ist deutlich erkennbar, dass die exakte Position des Partikels nicht genau determinierbar ist, da im aufgenommenen Bild die Umgebung des Partikels jeweils als unscharfe Wolke erscheint, die auf starke Feldstörungen aufgrund des Partikels zurückzuführen ist.

Die gestellte Aufgabe einer schnellen und exakten Positionsbestimmung des zu untersuchenden Partikels wird nun dahingehend gelöst, dass mittels einer der unterschiedlichen Messmethoden eine eindimensionale Projektion in jeder Raumrichtung erzeugt wird, mit der das Partikel präzise lokalisiert werden kann. Diese Methode funktioniert auch dann, wenn das Partikel selbst keine perfekte symmetrische Form hat (z.B. rund oder elliptisch), da die Spins in der gesamten Umgebung des Partikels in der Projektion symmetrisch verschoben erscheinen.

Folgende Beispiele, die sowohl mit Hilfe von Spin-Echo Sequenzen als auch mittels Gradientenechos erstellt wurden, belegen dieses erfindungsgemäße Prinzip:

### Erfindungsgemäße Messsequenzen

### Beispiel 1: Spin-Echo (MSME):

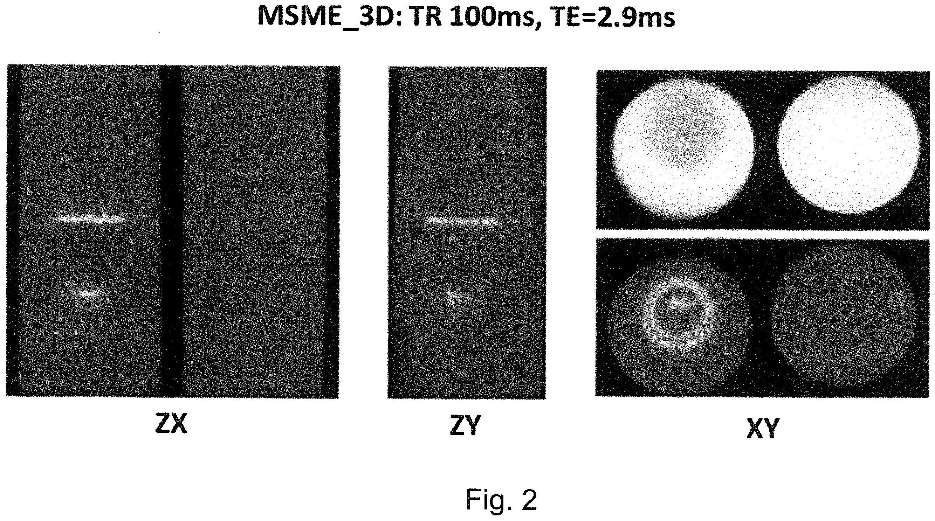

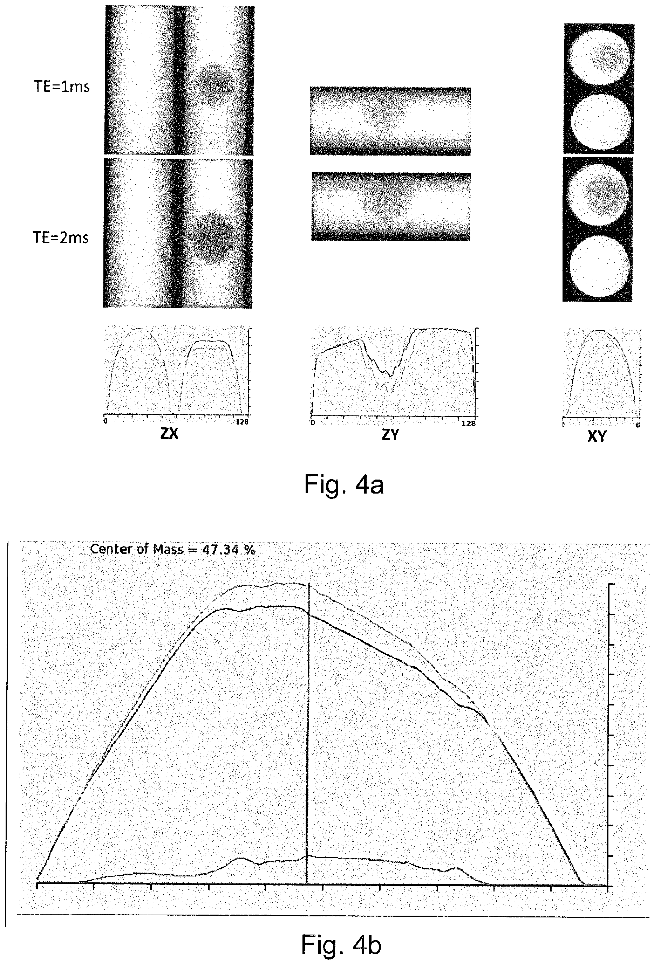

In Fig. 2 ist zu erkennen, welchen Effekt auf die MR-Bildgebung das magnetische Partikel in einer Spin-Echo Methode (MSME) hat. Quer zum B₀-Feld, also in der ZX- und in der ZY-Ebene ist jeweils ein heller Streifen zu erkennen, der etwas vom Zentrum des Artefakts entfernt liegt. In der XY-Ebene (in Richtung des B₀ Felds gesehen) ist ein heller Ring zu erkennen, der sich symmetrisch um das Zentrum des Partikels ausdehnt. Die Aufnahme wurde mit einer Echozeit TE=2,9ms gemacht. Der Durchmesser des Ringartefakts in XY-Ebene ändert sich mit der Echozeit. Also kann man beispielsweise auch zwei Messungen mit unterschiedlicher TE machen und darauf basierend die Projektionen nutzen zum Berechnen der exakten Position des Partikels. (Siehe Fig. 4)

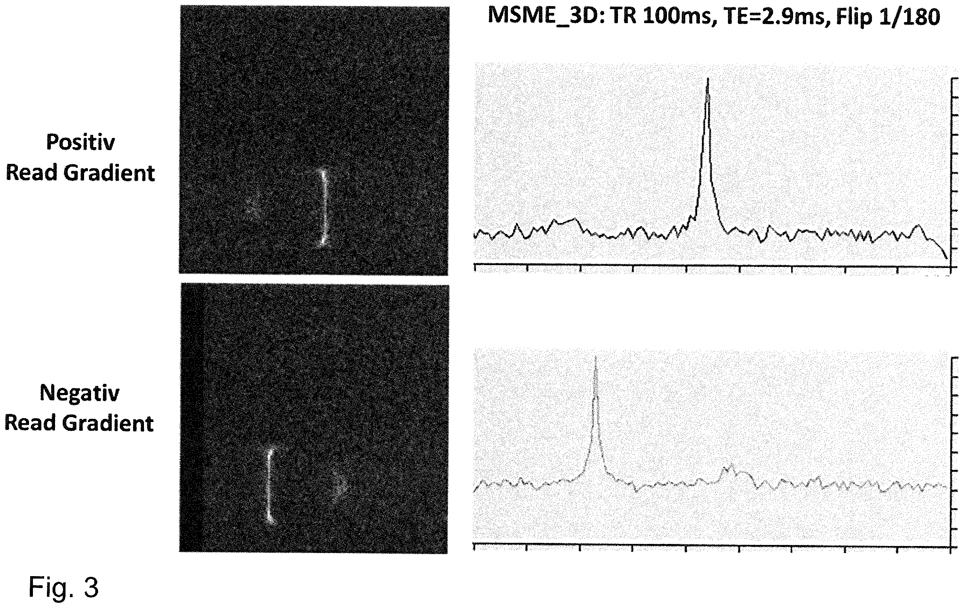

In den Ebenen senkrecht zum B₀-Feld ist die Bestimmung nicht eindeutig, da die Projektion asymmetrisch ist. In diesem Falle bedient man sich der zusätzlichen Schaltung eines Lesegradienten, welcher einmal mit positivem und einmal mit negativem Vorzeichen geschaltet wird. In Fig. 3 ist zu erkennen, dass man zwei sehr scharfe Projektionen erhält, die äquidistant zu einem Zentrum sind, an welchem sich das Magnetpartikel befindet.

Die Spin-Echo-Methode hat den Nachteil, dass eine relativ hohe Energie mit dem Rf Puls eingestrahlt wird. Für die Aufnahme der Projektion wie in den Beispielaufnahmen in Fig. 3 gezeigt, ist jedoch kein voller 180° Impuls erforderlich, auch bei Flipwinkeln von weniger als 90° ist eine Projektion messbar, jedoch in Abhängigkeit der Größe des Partikels. Die vorliegenden Aufnahmen wurden durch einen 1° Flipwinkel erstellt, wobei das kleinere Partikel mit 0,35mm Durchmesser nur schwer auszumachen ist. Die sichtbaren Aufnahmen beziehen sich auf das Partikel mit dem größeren Durchmesser von 1 mm.

### Beispiel 2: Änderung der Echozeit (Gradientenecho):

In **Fig. 4a** ist zu erkennen, dass das Artefakt mit der Gradientenechozeit an Breite zunimmt und zwar in jeder der drei Raumebenen. Die Echozeit bewirkt eine symmetrische Ausbreitung der Störung. Diesen Effekt kann man sich zunutze machen, um auf die genaue Position des Partikels zurückzuschließen.

Die Position eines Magnetpartikels wird beispielsweise ermittelt, indem man das Massenzentrum der Differenz beider Projektionen bestimmt: **Fig. 4b** zeigt eine Projektion mit TE=1ms (oberste Kurve), eine Projektion mit TE=2ms (mittlere Kurve) und die Differenzfunktion (unterste Kurve). Das ermittelte Massenzentrum lag hier bei 47.34% der Projektionsrichtung. Bei einer Projektion von 10cm wäre somit der Magnetpartikel 4,734cm vom linken Rand entfernt bzw. 2,66cm links der Mitte.

### Beispiel 3: Änderung der Sweepwidth (Gradientenecho):

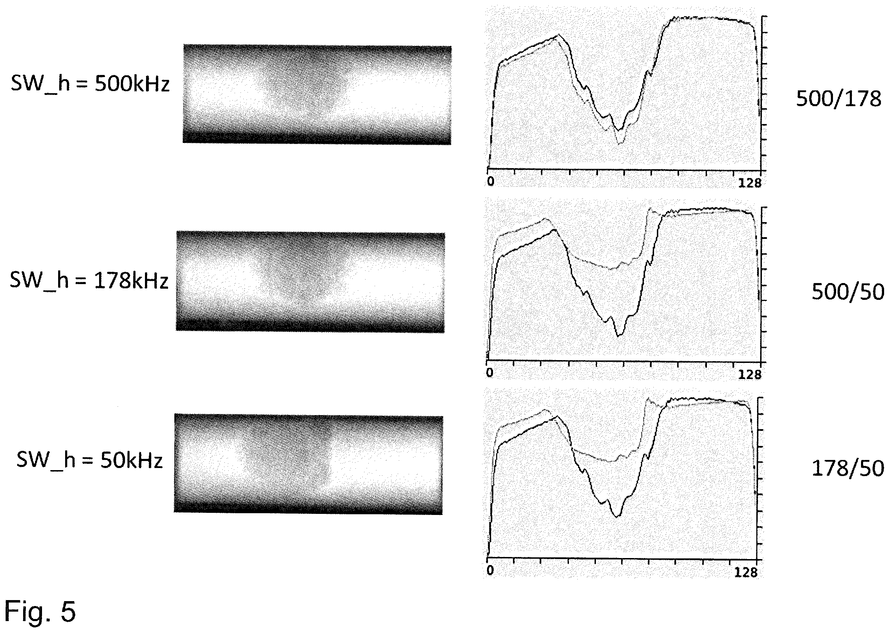

Mit der Änderung der Sweepwidth (Bandbreite der Digitalisierung) geht eine Änderung der Stärke des Lesegradienten einher, sodass der Abbildungsbereich (Field Of View) unverändert bleibt. **Fig. 5** zeigt, dass bei unterschiedlichen Digitalisierungszeiten die Störung durch das Magnetpartikel im Bild unsymmetrisch wird. Die Störung wird deformiert und auch vom Mittelpunkt weg verschoben. Zur Auswertung dieses Messartefakts hinsichtlich der Positionsbestimmung kann eine Mustererkennung verwendet werden, bei welcher mehrere Projektionen mit unterschiedlichen Bandbreiten herangezogen werden. Hierbei werden beispielsweise einmalig Kalibrierungsmessungen durchgeführt, bei denen die charakteristischen Störungen, deren Differenzfunktion sowie die dazugehörigen Abweichung der Partikelposition zum Massenzentrum der Differenzfunktion ermittelt werden.

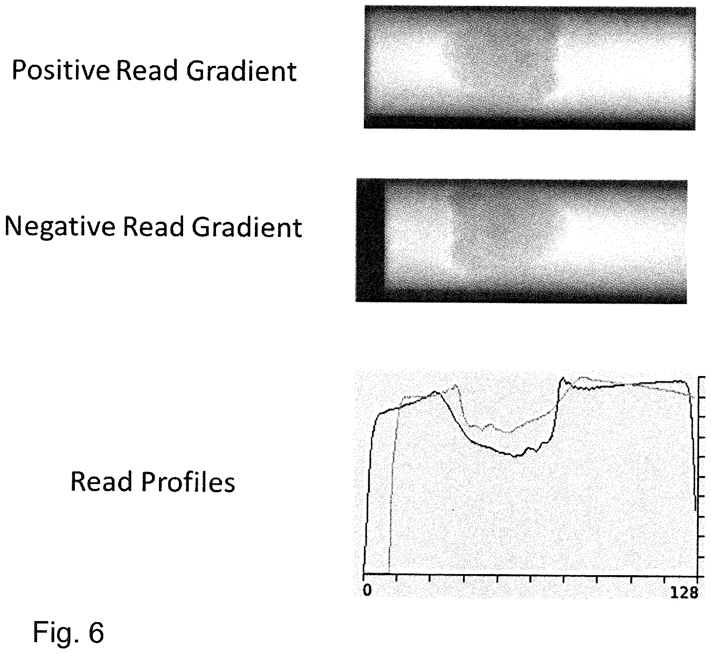

Die Asymmetrie, die beispielsweise in Fig. 5 bei 50kHz erkennbar ist, kann auch wiederum genutzt werden, um ähnlich wie im obigen Beispiel 1 den Lesegradienten umzukehren, sodass aus der jeweils entgegengesetzten Projektion von positiver und negativer Gradientenschaltung eine eindeutige Position zu lokalisieren ist, wie in **Fig. 6** zu erkennen ist, die die Lokalisierung eines Magnetpartikels unter Ausnutzung eines asymmetrischen Artefakts mit Hilfe entgegengesetzter Lesegradienten zeigt.

Die in den Figuren der Zeichnung gezeigten Beispielaufnahmen von Magnetpartikeln sind in einem homogenen Milieu, genauer in einem Gel erstellt worden. Somit stehen die 1H Spins in Umgebung des Magnetpartikels unter gleichem Einfluss der Nachbarspins. Anders verhält es sich bei der Bildgebung in einem nicht homogenen Objekt, wie z.B. im Körper von Tier und Mensch. In unterschiedlichen Geweben ändern sich die Spin-Spin und die Spin-Gitter Relaxationszeiten und die 1H Kerndichten sind sehr variabel. Als Extrembeispiel wäre ein signalfreier Raum in einer Gasblase. Wäre das Partikel in dieser Position, so wäre es nur schwerlich lokalisierbar. Auch die Inhomogenität der Gewebe erschwert die Lokalisierung mit der genannten Methode. In einer bevorzugten Ausführung kann das Magnetpartikel mit einem anderen NMR-aktiven Kern markiert werden. Ein solches Tracermaterial können im Prinzip alle NMR-aktiven Kerne verwendet werden. Diese würden dann über die entsprechende Frequenz ausgelesen, sodass es immer dieselben Störbilder ergibt, unabhängig von den benachbarten Gewebestrukturen.

Beispielhaft können hier sämtliche spinaktiven Kerne als Tracermaterial verwendet werden (6Li, 10B, 14N, 15N, 17O, 19F, 23Na, 29Si, 31P, 35Cl, 113Cd, 129Xe, 195Pt), bevorzugt werden jedoch solche, die als Heteroatome in Kunststoffen zu finden sind, wie F in Teflon, Cl in PVC, N in Polyurethanen, etc.

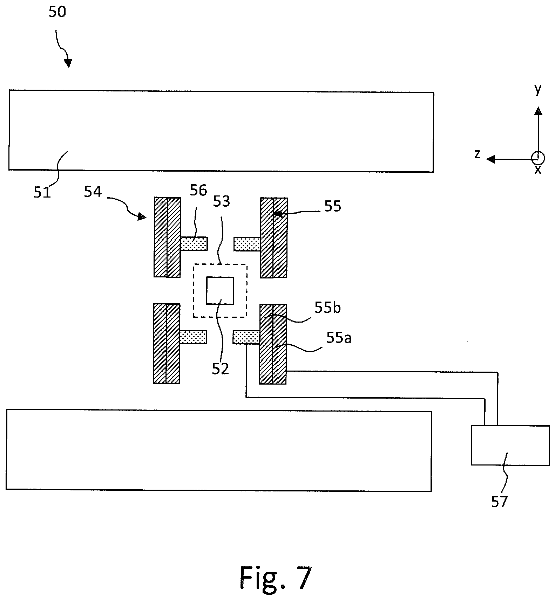

**Fig. 7** schließlich zeigt eine Ausführungsform eines erfindungsgemäßen MRI-Systems 50, insbesondere zur Durchführung einer Positionsbestimmung und/oder Positionsänderung eines ferromagnetischen Partikels (Objekts) im Rahmen der vorliegenden Erfindung.

Das MRI-System 50 weist einen Magneten (Hauptmagnetspulensystem, typischerweise supraleitend) 51 auf, mit dem in einem Messvolumen 52 ein starkes, homogenes Magnetfeld B₀ in horizontale z-Richtung erzeugt werden kann, typischerweise von einer Stärke von 1T oder mehr. Mit einem Hochfrequenz-Anregungs- und Lesespulensystem 53 können RF-Pulse in das Messvolumen 52 eingestrahlt werden und ein RF-Signal des Messvolumens ausgelesen werden. Weiterhin ist ein Gradientenspulensystem 54 vorgesehen, mit dem Magnetfeldgradienten im Messvolumen 52 erzeugt werden können.

Das Gradientenspulensystem 54 umfasst hier ein erstes Spulensubsystem 55, mit dem Magnetfeldgradienten in einer vertikalen y-Richtung erzeugt werden können, d.h. die Magnetfeldstärke ändert sich entlang y-Richtung. Weiterhin ist ein zweites Spulensubsystem 56 vorgesehen, mit dem Magnetfeldgradienten in der horizontalen z-Richtung erzeugt werden können, d.h. die Magnetfeldstärke ändert sich entlang der z-Richtung.

Das Gradientenspulensystem 54 wird über eine elektronische Steuereinrichtung 57 gesteuert.

Das zu untersuchende ferromagnetische Partikel -oder auch eine Vielzahl derartiger Partikel in einem Bündel- kann als Mikromaschine, Mikroroboter oder Mikrokomponente ausgebildet sein. Eine typische Größe eines solchen ferromagnetischen Partikels kann von 0,02mm bis zu wenigen Millimetern betragen (bezogen auf den größten Durchmesser). Das ferromagnetische Partikel besteht teilweise oder vollständig aus ferromagnetischem Material, insbesondere Eisen, Kobalt oder Nickel oder Legierungen dieser Metalle.

### Bezugszeichenliste

- 50: MRI-System
- 51: Magnet
- 52: Messvolumen
- 53: Hochfrequenz-Anregungs- und Lesespulensystem
- 54: Gradientenspulensystem
- 55: erstes Spulensubsystem
- 55a: Hauptteil
- 55b: Zusatzteil
- 56: zweites Spulensubsystem
- 57: Steuereinrichtung

### Referenzliste

[1] US 7,962,194 B2
[2] US 8,948,841 B2
[3] Evert-jan P. A. Vonken et al, "Direct in vitro comparison of six 3D positive contrast methods for susceptibility marker imaging", J Magn Reson Imaging. 2013 August; 38(2): 344-357
[4] Matthias Stuber et al, "Positive Contrast Visualization of Iron Oxide-Labeled Stem Cells using Inversion-Recovery With ON-Resonant Water Suppression (IRON)", Magnetic Resonance in Medicine 58:1072-1077 (2007)

## Patentansprüche

1. Verfahren zur Bestimmung der Position wenigstens eines ferromagnetischen Partikels in einem Objekt mittels eines MRI-Systems (50),
wobei eine MRI-Messsequenz auf ein Messvolumen (52) angewandt wird, in welchem sich das Partikel befindet,
wobei mindestens zwei Projektionen pro Raumrichtung aufgenommen werden, und wobei jeweils ein Lesegradient zur Aufnahme jeweils einer eindimensionalen Projektion geschaltet wird,
**dadurch gekennzeichnet,**
**dass** für die beiden Projektionen ein Parameter vorgewählt und dessen Wert bei der zweiten Projektion gegenüber der ersten geändert wird,
**dass** als zu ändernder Parameter eine Echo-Zeit oder eine Amplitude des Lesegradienten vorgewählt wird, wobei im Falle der Änderung der Amplitude des Lesegradienten das Field of View beibehalten wird, und dass mit Hilfe der beiden Projektionen die räumliche Position des ferromagnetischen Partikels ermittelt wird.

2. Verfahren nach einem der vorhergehenden Ansprüche, **dadurch gekennzeichnet, dass** jeweils zwei 1dimensionale Projektionsbilder in drei unterschiedlichen, vorzugsweise zueinander senkrechten, Raumrichtungen aufgenommen werden und daraus die räumliche Position des ferromagnetischen Partikels im 3dimensionalen Raum bestimmt wird.

3. Verfahren nach einem der vorhergehenden Ansprüche, **dadurch gekennzeichnet, dass** die Position des ferromagnetischen Partikels durch Differenzbildung der aufgenommenen 1dimensionalen Projektionsbilder oder durch Übereinanderlegen der aufgenommenen 1 dimensionalen Projektionsbilder oder mittels Mustererkennung im Vergleich der aufgenommenen 1 dimensionalen Projektionsbilder miteinander bestimmt wird.

4. Verfahren nach einem der vorhergehenden Ansprüche, **dadurch gekennzeichnet, dass** das ferromagnetische Partikel mit einem anderen NMR-aktiven Kern mit einer anderen Frequenz, die nicht identisch mit der Protonenfrequenz ist, markiert wird.

5. Verfahren nach Anspruch 4, **dadurch gekennzeichnet, dass** als Markiermaterial spinaktive Kerne verwendet werden, vorzugsweise solche, die als Heteroatome in Kunststoffen zu finden sind, wie F in Teflon, Cl in PVC, N in Polyurethanen, insbesondere 6Li, 10B, 14N, 15N, 17O, 19F, 23Na, 29Si, 31P, 35Cl, 113Cd, 195Pt.

6. MRI-System (50), umfassend einen Magneten (51) zur Erzeugung eines homogenen NMR-Magnetfelds B₀ in einem Messvolumen (52), ein Gradientenspulensystem (54) zur Erzeugung von Magnetfeldgradienten im Messvolumen (52), und ein Hochfrequenz-Anregungs- und Lesespulensystem (53) zum Einstrahlen von Hochfrequenz-Pulsen in das Messvolumen (52) und zum Auslesen des Messvolumens (52), **dadurch gekennzeichnet, dass** das MRI-System (50) eingerichtet ist zum Bestimmen der Position eines ferromagnetischen Partikels mittels eines Verfahrens nach einem der Ansprüche 1 bis 3.

## Claims

1. Method for determining the position of at least one ferromagnetic particle in an object by means of an MRI system (50),
wherein an MRI measurement sequence is applied to a measurement volume (52), in which the particle is situated,
wherein at least two projections are recorded per spatial direction, and
wherein a read gradient is switched in each case for recording a one-dimensional projection in each case,
**characterized in that**
a parameter is preselected for each of the two projections and its value in the second projection is changed with respect to the first,
that an echo time or an amplitude of the read gradient is preselected as parameter to be changed, wherein in case the amplitude of the read gradient is changed, the field of view is maintained, and
that the spatial position of the ferromagnetic particle is ascertained with the aid of the two projections.

2. Method according to any one of the preceding claims, **characterized in that** two one-dimensional projection images are recorded in each case in three different, preferably orthogonal, spatial directions and the spatial position of the ferromagnetic particle in the three-dimensional space is determined therefrom.

3. Method according to any one of the preceding claims, **characterized in that** the position of the ferromagnetic particle is determined by forming the difference of the recorded one-dimensional projection images or by overlaying the recorded one-dimensional projection images or by means of pattern recognition when comparing the recorded one-dimensional projection images to one another.

4. Method according to any one of the preceding claims, **characterized in that** the ferromagnetic particle is marked with another NMR-active nucleus with another frequency which is not identical to the proton frequency.

5. Method according to claim 4, **characterized in that** spin-active nuclei are used as marker material, preferably those that can be found as heteroatoms in plastic materials, such as F in Teflon, Cl in PVC, N in polyurethanes, in particular 6Li, 10B, 14N, 15N, 170, 19F, 23Na, 29Si, 31P, 35CI, 113Cd, 195Pt.

6. MRI system (50), comprising a magnet (51) for producing a homogeneous NMR magnetic field B₀ in a measurement volume (52), a gradient coil system (54) for producing magnetic field gradients in the measurement volume (52), and a radiofrequency excitation and read coil system (53) for radiating radiofrequency pulses into the measurement volume (52) and for reading the measurement volume (52), **characterized in that** the MRI system (50) is configured to determine the position of a ferromagnetic particle by means of a method according to any one of Claims 1 to 3.

## Revendications

1. Procédé de définition de la position d'au moins une particule ferromagnétique dans un objet au moyen d'un système d'IRM (50),
dans lequel une séquence de mesure IRM est appliquée sur un volume de mesure (52) à l'intérieur duquel se trouve la particule,
dans lequel au moins deux projections par direction spatiale sont capturées et dans lequel un gradient de lecture respectif est commuté pour capturer respectivement une projection unidimensionnelle,
**caractérisé**
**en ce que** pour les deux projections un paramètre est présélectionné et la valeur de celui-ci lors de la deuxième projection est modifiée par rapport à la première,
**en ce qu'**un temps d'écho ou une amplitude du gradient de lecture est présélectionné(e) en tant que paramètre à modifier, dans lequel le champ visuel est conservé dans le cas de la modification de l'amplitude du gradient de lecture,
et **en ce que** la position spatiale de la particule ferromagnétique est déterminée à l'aide des deux projections.

2. Procédé selon une des revendications précédentes, **caractérisé en ce que** deux images projetées unidimensionnelles respectives sont capturées dans trois directions spatiales différentes, de préférence perpendiculaires l'une par rapport à l'autre et la position spatiale de la particule ferromagnétique est définie dans l'espace tridimensionnel à partir de celles-ci.

3. Procédé selon une des revendications précédentes, **caractérisé en ce que** la position de la particule ferromagnétique est définie par différenciation des images projetées unidimensionnelles capturées ou par superposition des images projetées unidimensionnelles capturées ou au moyen d'une reconnaissance de motif lors de la comparaison des images projetées unidimensionnelles capturées l'une par rapport à l'autre.

4. Procédé selon une des revendications précédentes, **caractérisé en ce que** la particule ferromagnétique est marquée avec un autre noyau actif en RMN doté d'une autre fréquence qui n'est pas identique à la fréquence du proton.

5. Procédé selon la revendication 4, **caractérisé en ce que** des noyaux à spin actif sont utilisés comme matériau de marquage, de préférence de tels noyaux qui se trouvent comme hétéroatomes dans les plastiques, comme F pour le Téflon, Cl dans le PVC, N pour les polyuréthanes, en particulier 6Li, 10B, 14N, 15N, 17O, 19F, 23Na, 29Si, 31P, 35Cl, 113Cd, 195Pt.

6. Système d'IRM (50), comprenant un aimant (51) permettant de produire un champ magnétique homogène de RMN B₀ dans un volume de mesure (52), un système de bobines de gradient (54) permettant de produire des gradients de champ magnétique dans le volume de mesure (52), et un système d'impulsions haute fréquence d'excitation et de lecture (53) permettant de rayonner des impulsions haute fréquence dans le volume de mesure (52) et de lire le volume de mesure (52), **caractérisé en ce que** le système d'IRM (50) est configuré afin de définir la position d'une particule ferromagnétique au moyen d'un procédé selon une des revendications 1 à 3.
